(19) Europäisches Patentamt
European Patent Office
Office européen des brevets

(11) Numéro de publication : 0 211 775 B1

(12) # FASCICULE DE BREVET EUROPEEN

(45) Date de publication du fascicule du brevet : **27.02.91 Bulletin 91/09**

(51) Int. Cl.$^5$ : **C07C 205/20**, C07C 205/26, C07C 215/74, C07C 201/06

(21) Numéro de dépôt : **86420164.5**

(22) Date de dépôt : **24.06.86**

(54) **Procédé de nitration de composés phénoliques.**

(30) Priorité : **05.07.85 FR 8510520**

(43) Date de publication de la demande : **25.02.87 Bulletin 87/09**

(45) Mention de la délivrance du brevet : **27.02.91 Bulletin 91/09**

(84) Etats contractants désignés : **BE CH DE FR GB IT LI NL**

(56) Documents cités :
**EP-A- 0 071 279**
**FR-A- 1 452 911**
**FR-A- 1 551 106**
**US-A- 2 868 844**
**US-A- 3 694 513**

(56) Documents cités :
**CHEMICAL ABSTRACTS, vol. 77, no. 9, 28 août 1972, page 464, résumé no. 61541p, Columbus, Ohio, US**
**CHEMISCHE BERICHTE, vol. 106, no. 3, 1973, pages 727-733; B. EISTERT et al.: "Umsetzungen von Diazoalkanen mit 2,6-Dichlor-p-benzochinon"**
**Advanced Organic Chemistry, 1985, p. 246-248 (Jerry March), John Willey & Sons, New York**

(73) Titulaire : **RHONE-POULENC CHIMIE**
**25, quai Paul Doumer**
**F-92408 Courbevoie Cédex (FR)**

(72) Inventeur : **Ratton, Serge**
**Hameau de Belmont Vaulx-Milieu**
**F-38090 Villefontaine (FR)**

(74) Mandataire : **Vignally, Noel et al**
**RHONE-POULENC INTERSERVICES Service Brevets Chimie Centre de Recherches de Saint-Fons B.P. 62**
**F-69192 Saint-Fons Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 211 775 B1

Jouve, 18, rue Saint-Denis, 75001 PARIS

**Description**

La présente invention concerne un procédé de nitration de composés phénoliques.

Il est connu de nitrer de nombreux composés, soit très fréquemment à l'aide d'un mélange acide sulfurique/acide nitrique, soit à l'aide d'une solution diluée ou concentrée d'acide nitrique.

C'est ainsi que l'on peut se reporter par exemples aux ouvrages généraux tels que par exemple "NITRATION AND AROMATIC REACTIVITY" de J.G. HOGGETT et R.B. MOODIE (1971) édité par Cambridge University Press et "AROMATIC NITRATION" de K. SCHOFIELD (1980) édité par Cambridge University Press.

Les nitrophénols (essentiellement les isomères ortho et para) sont généralement préparés par nitration du phénol, par l'acide nitrique et en milieu sulfurique. On peut se référer par exemple à l'article de R.B. MOODIE dans "Journal Of Chemical Society, Perkin Trans." II, 1985, page 467.

La nitration des phénols est le plus souvent réalisée en milieu sulfurique et rarement en milieu organique.

Lors de la préparation des nitrophénols, on observe fréquemment des sous-produits d'oxydation, tels que notamment des dérivés de la benzoquinone et des dérivés de masse moléculaire élevée provenant de la condensation de plusieurs molécules aromatiques, ce qui nécessite de délicates opérations de purification.

Le brevet US-A-2 868 844 décrit la nitration en milieu solvant aprotique apolaire, non miscible à l'eau, de dialkyl-2,6 phénol, par l'acide nitrique à 30 à 70% de concentration avec un rapport molaire global acide/dialkylphénol de 1 à 2 environ.

Le brevet EP-A-0 071 279 décrit dans son exemple 4 la nitration du diéthyl-2,6 phénol dans le chloroforme par de l'acide nitrique à 70% avec un rapport molaire acide/diéthyl phénol de 1.

Le résumé des chemical abstracts 1972, 77 n° 61541p décrit la nitration du dichloro-2,4 phénol en dichloro-2,4 nitro-6 phénol.

En poursuivant les recherches, il a été trouvé que l'on peut également nitrer le dichloro-2,6 phénol, dont les substituants C1 ont un effet inductif inverse de celui des groupements alkyles comme on peut le voir dans "Advanced Organic Chemistry" de Jerry March, tableau 5 page 247 (Mc GRAW-HILL BOOK COMPANY, édition de 1985).

Plus précisément, la présente invention consiste tout d'abord en un procédé de nitration d'un composé phénolique de formule (I) :

(I)

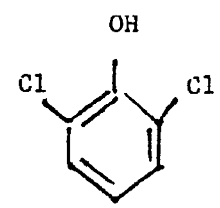

caractérisé en ce que ledit composé phénolique est introduit dans une solution aqueuse d'acide nitrique ayant une concentration en poids de 10% à 70% et en ce que le rapport molaire global acide nitrique/composéphénolique (I) est compris entre 10 et 1,2.

Le mode de mise en contact des réactifs peut se faire de diverses manières.

On peut introduire le composé phénolique de formule (I) sous forme solide dans la solution aqueuse d'acide nitrique.

On peut accélérer la réaction de nitration en introduisant de faibles quantités de nitrite de sodium ou de vapeurs nitreuses.

Le nitrophénol substitué formé, qui est pratiquement insoluble dans l'eau, précipite et peut être ainsi isolé.

Cette variante du procédé, bien qu'elle conduise à un bon rendement en nitrophénol substitué, présente l'inconvénient de fournir un nitrophénol substitué renfermant les impuretés que sont les sous-produits de la réaction.

Parmi ces impuretés se trouvent notamment les dérivés de la benzoquinone, provenant de l'oxydation du composé phénolique, qui colorent le nitrophénol substitué.

C'est pour pallier cet inconvénient que de préférence on met en oeuvre le composé phénolique (I) sous la forme d'une solution dans un solvant organique aprotique apolaire non miscible à l'eau.

Il est bien évident que le solvant aprotique apolaire, utilisé dans l'un ou l'autre des procédés de l'invention, sera tel qu'il soit stable vis-à-vis de l'acide nitrique dans les conditions de la nitration.

Le solvant aprotique apolaire est essentiellement un hydrocarbure aliphatique, un hydrocarbure cycloaliphatique, un hydrocarbure aromatique, un hydrocarbure aliphatique chloré, un hydrocarbure cycloaliphatique chloré ou un hydrocarbure aromatique chloré.

Afin d'obtenir un nitrophénol substitué de bonne pureté et d'isolement facile, le solvant aprotique apolaire est choisi de telle façon qu'il ne dissolve pratiquement pas ou très peu ledit nitrophénol substitué, mais que par contre il dissolve bien le dérivé de la benzoquinone, qui se forme au cours de la réaction.

On choisira donc également la quantité de solvant utilisée de manière à dissoudre suffisamment les sous-produits formés et plus particulièrement le dérivé de la benzoquinone.

Comme hydrocarbure aliphatique, on peut citer notamment l'hexane, l'heptane, le nonane, le décane

et le dodécane.

Comme hydrocarbure cycloaliphatique, on peut citer notamment le cyclohexane, le méthyl-cyclohexane, l'éthylcyclohexane, le tertiobutylcyclohexane, le diméthyl-1,1 cyclohexane, le diméthyl-1,2 cyclohexane, le propylcyclohexane, l'isopropylcyclohexane.

Comme hydrocarbure aromatique, on peut citer notamment le benzène, le toluène, l'orthoxylène, le métaxylène, le paraxylène, l'éthylbenzène, l'isopropylbenzène.

Comme hydrocarbure aliphatique chloré, on peut citer notamment le tétrachlorure de carbone, le tétrachloroéthylène, l'hexachloroéthane, le chlorure de méthylène, le dichloroéthane, le tétrachloroéthane, le trichloroéthylène, le chloro-1 butane, le dichloro-1, 2 butane.

Comme hydrocarbure cycloaliphatique chloré, on peut citer notamment le chlorocyclohexane.

Comme hydrocarbure aromatique chloré, on peut citer notamment le monochlorobenzène, le dichloro-1,2 benzène, le dichloro-1,3 benzène, le dichloro-1, 4 benzène.

Parmi ces familles de solvants aprotiques apolaires, on utilise de préférence les hydrocarbures aromatiques, les hydrocarbures aromatiques chlorés et les hydrocarbures aliphatiques chlorés.

Parmi ces solvants aprotiques apolaires préférés, on utilise tout particulièrement le tétrachlorure de carbone, le toluène, le benzène, le monochlorobenzène.

La concentration pondérale initiale de la solution aqueuse d'acide nitrique utilisée dans la présente invention sera de préférence de 20% à 50%.

La température à laquelle sont mis en oeuvre les procédés peut varier dans de larges limites, par exemple de 0°C à 100°C. De préférence on opèrera plutôt entre 20°C et 60°C.

Les procédés de l'invention peuvent être mis en oeuvre de manière discontinue ou de manière continue.

Lorsque l'on opère en discontinu la durée totale de la réaction peut être variable. Généralement la durée de l'introduction du composé phénolique varie de 15 minutes à plusieurs heures. Le plus souvent elle se situe entre 30 minutes et 4 heures.

L'invention permet le recyclage aisé des solvants et réactifs non consommés. Ainsi lorsque l'on utilise un solvant aprotique apolaire pour dissoudre le composé phénolique (I) et le faire réagir avec la solution aqueuse d'acide nitrique, ce solvant peut être directement recyclé après une purge permettant d'éliminer une partie des sous-produits. La solution d'acide nitrique peut également servir à nouveau, après une purge permettant d'éliminer l'eau formée et après rajout d'une quantité d'acide nitrique égale à celle qui a été consommée.

Parmi les composés phénoliques de formule (I), les procédés de l'invention sont plus particulièrement applicables de manière très intéressante au dichloro-2,6 phénol pour préparer le dichloro-2,6 nitro-4 phénol. En effet ce dernier composé est un intermédiaire important qui permet d'obtenir :

- par hydrogénation catalytique, par exemple en présence d'un métal noble ou de nickel de Raney, ou par hydrogénation chimique, par exemple à l'aide de fer + acide chlorhydrique, l'amino-4 dichloro-2,6 phénol utilisé en agrochimie ;
- par méthylation de la fonction phénolique, le dichloro-2,6 nitro-4 anisole, qui est un intermédiaire utilisé dans le domaine pharmaceutique.

Le dichloro-2,6 nitro-4 phénol est habituellement préparé par chloration du nitro-4 phénol. Ce procédé n'est pas très satisfaisant car la dichloration est délicate à réaliser et l'on obtient, outre le dichloro-2,6 nitro-4 phénol, du dichloro-2,5 nitro-4 phénol, du chloro-2 nitro-4 phénol, du trichloro-2, 3, 6 nitro-4 phénol, en quantités plus ou moins importantes, qu'il est très difficile ensuite de séparer. D'autre part le nitro-4 phénol est une matière première relativement coûteuse.

Les procédés selon l'invention offrent donc une voie d'accès simple à des nitrophénols substitués de bonne pureté, avec des rendements élevés.

Lorsque les présents procédés sont appliqués au dichloro-2,6 phénol, un avantage supplémentaire réside dans la facilité d'enchaînement de la nitration dudit dichloro-2,6 phénol, avec la propre synthèse du dichloro-2,6 phénol à partir du chloro-2 phénol.

En effet un procédé de chloration sélective du chloro-2 phénol en dichloro-2,6 phénol, par le chlore gazeux, consiste à opérer à une température généralement comprise entre 40°C et 120°C, dans un solvant aprotique apolaire et en présence de 0,001% à 0,100% en poids d'une amine primaire, secondaire ou tertiaire par rapport au dit solvant. Le solvant aprotique apolaire utilisé dans la chloration du chloro-2 phénol peut être par exemple le tétrachlorure de carbone, le tétrachloroéthylène ou le monochlorobenzène, qui conviennent également très bien pour réaliser les présents procédés de nitration.

L'amine servant dans la chloration du chloro-2 phénol peut être plus particulièrement la diisopropylamine, la tertiobutylamine ou l'aniline.

Ce procédé de chloration étant sélectif, on peut en pratique utiliser directement la solution finale ainsi obtenue de dichloro-2,6 phénol dans le solvant aprotique apolaire approprié pour la nitration par la solution aqueuse d'acide nitrique et ainsi passer très commodément du chloro-2 phénol, qui est un produit industriel courant, au dichloro-2,6 nitro-4 phénol qui est un intermédiaire très important.

La présente invention s'applique également de manière particulièrement intéressante au dichloro-2,6 phénol lorsqu'il est en mélange avec d'autres composés, notamment avec le trichloro-2,4,6 phénol.

On peut ainsi valoriser un mélange industriel brut.

Il est bien évident que les procédés de l'invention demeurent très intéressants, lorsqu'ils sont appliqués à d'autres composés phénoliques de formule (I), notamment à ceux qui ont été cités plus particulièrement.

Les exemples qui suivent ont pour but d'illustrer la présente invention.

EXEMPLE 1

Dans un ballon en verre de 4000 cm³, équipé d'un thermomètre, d'un agitateur central, d'un réfrigérant et d'une ampoule de coulée, on charge 1535 cm³ de solution aqueuse d'acide nitrique à 40% en poids par poids.

Dans l'ampoule de coulée, on charge une solution de dichloro-2,6 phénol dans du tétrachlorure de carbone (200 g de dichloro-2,6 phénol dissous dans 1230 cm³ de tétrachlorure de carbone).

On chauffe la solution d'acide nitrique à 35°C à l'aide d'un bain-marie ; ensuite on coule la solution de dichloro-2,6 phénol en 1 h 30 min.

Pendant la coulée, on constate dans la masse réactionnelle une démixtion entre la couche aqueuse d'acide nitrique et le tétrachlorure de carbone, l'apparition d'un précipité et un faible dégagement de vapeurs rousses.

A la fin de la coulée, on continue l'agitation pendant 5 minutes.

Ensuite on refroidit à environ 4°C, à l'aide de glace. On essore le précipité sur un filtre en verre fritté, on le lave avec deux fois 100 cm³ d'eau glacée, puis on le sèche sous vide dans un dessicateur.

On récupère ainsi 229 g de dichloro-2,6 nitro-4 phénol pur (sans sous-produits et notamment sans dichloro-2,6 benzoquinone) contenant 1,3 % d'eau.

Rendement en produit isolé par rapport au dichloro-2,6 phénol chargé (RR) : 87%.

Le dichloro-2,6 nitro-4 phénol est une poudre jaune pâle.

On sépare la phase organique et la phase aqueuse par décantation ; on dose dans chaque phase, par chromatographie en phase liquide haute performance, le dichloro-2,6 nitro-4 phénol dissous et la dichloro-2, 6 benzoquinone.

RR total en dichloro-2,6 nitro-4 phénol : 90,2% (le dichloro-2,6 nitro-4 phénol isolé ne contient pas de dichloro-2,6 benzoquinone).

RR en dichloro-2,6 benzoquinone : 8,9%.

On note que la dichloro-2,6 benzoquinone est en majorité dans la phase organique.

EXEMPLE 2

Dans un ballon en verre de 250 cm³, équipé d'un thermomètre, d'un agitateur central, d'un réfrigérant et d'une ampoule de coulée, on charge 60 cm³ de solution aqueuse d'acide nitrique à 25% en poids par poids.

Dans l'ampoule de coulée, on charge une solution de dichloro-2,6 phénol dans du tétrachlorure de carbone (8,15 g de dichloro-2,6 phénol dissous dans 50 cm³ de tétrachlorure de carbone).

On chauffe la solution d'acide nitrique à 35°C à l'aide d'un bain-marie ; ensuite on coule la solution de dichloro-2,6 phénol en 30 minutes.

Pendant la coulée, on constate dans la masse réactionnelle une démixtion entre la couche aqueuse d'acide nitrique et le tétrachlorure de carbone, l'apparition d'un précipité et un faible dégagement de vapeurs rousses.

A la fin de la coulée, on continue l'agitation pendant 5 minutes.

Ensuite on refroidit à environ 4°C, à l'aide de glace. On essore le précipité sur un filtre en verre fritté, on le lave avec deux fois 10 cm³ d'eau glacée, puis on le sèche sous vide dans un dessicateur.

Poids du produit isolé : 8,80 g (dont 8,70 g de dichloro-2,6 nitro-4 phénol pur).

Le produit isolé ne contient pas de dichloro-2,6 benzoquinone.

On sépare la phase organique et la phase aqueuse par décantation ; on dose dans chaque phase par chromatographie en phase liquide haute performance le dichloro-2,6 nitro-4 phénol et la dichloro-2,6 benzoquinone dissous.

RR total en dichloro-2,6 nitro-4 phénol : 86,1%.

RR en dichloro-2,6 benzoquinone : 9,8 %.

EXEMPLE 3

On répète l'exemple 2 en chargeant 60 cm³ de solution aqueuse d'acide nitrique à 35% en poids par poids. Le mode opératoire, la quantité de dichloro-2,6 phénol, le solvant et la durée de réaction sont les mêmes que dans l'exemple 2.

Poids du produit isolé : 8,75 g (dont 8,67 g de dichloro-2,6 nitro-4 phénol pur).

Le produit isolé ne contient pas de dichloro-2,6 benzoquinone.

RR total en dichloro-2,6 nitro-4 phénol : 86,8%.

RR en dichloro-2,6 benzoquinone : 8,0%.

EXEMPLE 4

On répète l'exemple 2 en chargeant 60 cm³ de solution aqueuse d'acide nitrique à 45% en poids par poids. Le mode opératoire, la quantité de dichloro-2,6 phénol, le solvant et la durée de réaction sont les mêmes que dans l'exemple 2.

Poids du produit isolé : 9,0 g (dont 8,82 g de dichloro-2,6 nitro-4 phénol pur).

Le produit isolé ne contient pas de dichloro-2,6 benzoquinone.

RR total en dichloro-2,6 nitro-4 phénol : 88,8%.

RR en dichloro-2,6 benzoquinone : 6,8%.

EXEMPLE 5

On répète l'exemple 2 en chargeant 60 $cm^3$ de solution aqueuse d'acide nitrique à 60% en poids par poids. Le mode opératoire, la quantité de dichloro-2,6 phénol, le solvant et la durée de réaction sont les mêmes que dans l'exemple 2.

Poids du produit isolé : 7,50 g (dont 7,35 g de dichloro-2,6 nitro-4 phénol pur).

Le produit isolé ne contient pas de dichloro-2,6 benzoquinone.

RR total en dichloro-2,6 nitro-4 phénol : 74,4%.

RR en dichloro-2,6 benzoquinone : 9,5%.

EXEMPLE 6

On répète l'exemple 2 selon le même mode opératoire et les mêmes durées de réaction mais avec les quantités suivantes de réactifs et de solvant :

– solution aqueuse d'acide nitrique à 35% en poids par poids : 60 $cm^3$

– solution de 8,15 g de dichloro-2,6 phénol dans 50 $cm^3$ de toluène. Poids du produit isolé : 7,95 g (dont 7,78 g de dichloro-2,6 nitro-4 phénol pur).

Le produit isolé ne contient pas de dichloro-2,6 benzoquinone.

RR total en dichloro-2,6 nitro-4 phénol : 88,9%.

RR en dichloro-2,6 benzoquinone : 10,6%.

EXEMPLE 7

On répète l'exemple 2 selon le même mode opératoire et les mêmes durées de réaction mais avec les quantités suivantes de réactifs et de solvant :

– solution aqueuse d'acide nitrique à 35% en poids par poids : 60 $cm^3$

– solution de 8,15 g de dichloro-2,6 phénol dans 50 $cm^3$ de monochlorobenzène.

Poids du produit isolé : 7,65 g (dont 7,21 g de dichloro-2,6 nitro-4 phénol pur).

Le produit isolé ne contient pas de dichloro-2,6 benzoquinone.

RR total en dichloro-2,6 nitro-4 phénol : 86,8%.

RR en dichloro-2,6 benzoquinone : 11,3%.

EXEMPLE 8

On répète l'exemple 2 selon le même mode opératoire et les mêmes durées de réaction mais avec les quantités suivantes de réactifs et de solvant :

– solution aqueuse d'acide nitrique à 35% en poids par poids : 60 $cm^3$

– solution de 8,15 g de dichloro-2,6 phénol dans 50 $cm^3$ de cyclohéxane.

Poids du produit isolé : 9,73 g (dont 9,10 g de dichloro-2,6 nitro-4 phénol pur et 0,39 g de dichloro-2,6 benzoquinone).

RR total en dichloro-2,6 nitro-4 phénol : 89,0%.

RR en dichloro-2,6 benzoquinone : 11,4%.

EXEMPLE 9

On répète l'exemple 2 selon le même mode opératoire et les mêmes durées de réaction mais avec les quantités suivantes de réactifs et de solvant :

– solution aqueuse d'acide nitrique à 45% en poids par poids : 14 g

– solution de 8,15 g de dichloro-2,6 phénol dans 25 $cm^3$ de tétrachlorure de carbone (solution saturée).

Poids du produit isolé : 9,62 g (dont 9,13 g de dichloro-2,6 nitro-4 phénol pur et 0,12 g de dichloro-2,6 benzoquinone).

RR total en dichloro-2,6 nitro-4 phénol : 89,4%.

RR en dichloro-2,6 benzoquinone : 12,2%.

EXEMPLE 10

Dans un ballon en verre de 125 $cm^3$, équipé d'un thermomètre, d'un agitateur central et d'un réfrigérant, on charge 25 $cm^3$ de solution aqueuse d'acide nitrique à 40% en poids par poids, que l'on porte à 35°C.

On introduit en 15 minutes environ 3,26 g de dichloro-2,6 phénol à l'aide d'une spatule. On n'observe pas de réaction immédiate. On ajoute quelques cristaux de nitrite de sodium afin de catalyser la réaction. On note une coloration jaune de la masse réactionnelle.

La réaction est lente ; on maintient pendant une nuit sous agitation à 35°C.

La masse réactionnelle contient alors un précipité jaune. On essore le précipité, on le lave par deux fois 5 $cm^3$ d'eau glacée et on le sèche sous vide dans un dessicateur.

RR en dichloro-2,6 nitro-4 phénol : 79,0%.

EXEMPLE 11

Dans l'appareillage décrit dans l'exemple 2, on charge :

– 20 g (122,7 mmoles) de dichloro-2,6 phénol,

– 130 $cm^3$ de tétrachlorure de carbone.

On chauffe à 35°C. On coule ensuite 29 g d'une solution aqueuse d'acide nitrique à 40% en poids (184 mmoles) en 20 minutes.

Au cours de la coulée la masse réactionnelle se colore rapidement en jaune ; il y a ensuite apparition d'un précipité jaune, une faible démixtion et un dégagement de vapeurs rousses.

En fin de coulée, on laisse agiter 15 minutes en température puis on refroidit à 0°C. On filtre, lave le précipité à l'eau, essore et sèche.

Poids du dichloro-2,6 nitro-4 phénol

isolé :22,64g.

Le produit isolé ne contient pas de dichloro-2,6 benzoquinone.

On sépare la phase organique et la phase aqueuse par décantation ; on dose dans chaque phase, par chromatographie en phase liquide haute performance, le dichloro-2,6 nitro-4 phénol dissous et la dichloro-2,6 benzoquinone.

On obtient les résultats suivants :

Taux de transformation (TT) du dichloro-2,6 phénol : 100%

RR en dichloro-2,6 nitro-4 phénol isolé : 88,7%

RR en dichloro-2,6 nitro-4 phénol total : 91,4%

RR en dichloro-2,6 benzoquinone : 8,2%.

## EXEMPLE 12

### Nitration d'un mélange de dichloro-2,6 phénol et de trichloro-2,4,6 phénol

Dans un appareillage similaire à celui de l'exemple 2, mais avec un réacteur de 500 $cm^3$, on charge :

- 36,8 g (225,76 mmoles) de dichloro-2,6 phénol,
- 3,68 g (18,64 mmoles) de trichloro-2,4,6 phénol,
- 360 g de tétrachlorure de carbone.

On obtient une solution à environ 10% en poids dans le tétrachlorure de carbone. On chauffe le mélange à 35°C sous agitation.

On coule 53,55 g d'une solution d'acide nitrique à 40% en poids en 15 minutes (338 mmoles : 1,5 fois la quantité molaire de dichloro-2,6 phénol).

Au cours de la coulée la masse réactionnelle se colore rapidement en jaune ; il y a ensuite apparition d'un précipité jaune, une faible démixtion et un dégagement de vapeurs rousses.

En fin de coulée le mélange est orangé ; on laisse agiter 15 minutes en température puis on refroidit à 0°C. On filtre, lave le précipité à l'eau, essore et sèche.

Poids du dichloro-2,6 nitro-4 phénol isolé : 41,70 g.

Le produit isolé ne contient pas de dichloro-2,6 benzoquinone.

On sépare la phase organique et la phase aqueuse par décantation ; on dose dans chaque phase, par chromatographie en phase liquide haute performance, le dichloro-2,6 nitro-4 phénol dissous et le trichloro-2,4,6 phénol qui n'a pas réagi.

On obtient les résultats suivants :

TT du dichloro-2,6 phénol : 100%

RR en dichloro-2,6 nitro-4 phénol isolé : 88,8%

RR en dichloro-2,6 nitro-4 phénol total : 91,5%.

On retrouve 2,1 g de trichloro-2,4,6 phénol qui n'ont pas réagi.

## EXEMPLE 13

### Nitration d'un mélange de dichloro-2,6 phénol et de trichloro-2,4,6 phénol

Dans un appareillage similaire à celui de l'exemple 2, mais avec un réacteur de 6 1, on charge 1293 g (8,4 moles) de solution aqueuse d'acide nitrique à 40,9% en poids.

On chauffe à 33°C sous agitation. A l'aide d'une pompe à piston on injecte en 2 h 50 min 3600 g (2280 $cm^3$) d'une solution de dichloro-2,6 phénol et de trichloro-2,4,6 phénol dans le tétrachlorure de carbone contenant :

- 2,098 moles de dichloro-2,6 phénol (9,5% en poids dans la solution),
- 0,169 moles de trichloro-2,4,6 phénol (0,93% en poids dans la solution).

On note un dégagement de vapeurs nitreuses tout au long de l'injection.

En fin d'injection on refroidit à 4°C, à l'aide de glace, en 55 minutes.

On filtre sur filtre Büchner ; on lave le précipité avec 700 $cm^3$ d'eau ; on essore et on sèche.

On obtient 366 g de dichloro-2,6 nitro-4 phénol (pas de dichloro-2,6 benzoquinone).

On sépare la phase organique et la phase aqueuse par décantation ; on dose dans chaque phase, par chromatographie en phase liquide haute performance, le dichloro-2,6 nitro-4 phénol dissous et la dichloro-2,6 benzoquinone.

On obtient les résultats suivants :

TT du dichloro-2,6 phénol : 100%

RR en dichloro-2,6 nitro-4 phénol isolé : 84,0%

RR en dichloro-2,6 nitro-4 phénol total : 87,3%.

RR en dichloro-2,6 benzoquinone : 9,4%.

## Revendications

1. Procédé de nitration du dichloro-2,6 phénol caractérisé en ce que ledit composé phénolique est introduit dans une solution aqueuse d'acide nitrique ayant une concentration en poids de 10 à 70% et en ce que le rapport molaire global acide nitrique/composé phénolique (I) soit compris entre 10 et 1,2.

2. Procédé selon l'une des revendications 1, caractérisé en ce que le composé phénolique est introduit sous forme solide dans la solution aqueuse d'acide nitrique.

3. Procédé selon l'une des revendications 1, caractérisé en ce que le composé phénolique est introduit sous la forme d'une solution dans un solvant aprotique apolaire, non miscible à l'eau.

4. Procédé selon la revendication 3, caractérisé en ce que le solvant aprotique apolaire est un hydrocarbure aliphatique, un hydrocarbure cycloaliphatique, un hydrocarbure aromatique, un hydrocarbure

aliphatique chloré, un hydrocarbure cycloaliphatique chloré ou un hydrocarbure aromatique chloré.

5. Procédé selon l'une des revendications 3 et 4, caractérisé en ce que le solvant aprotique apolaire est choisi parmi les hydrocarbures aromatiques, les hydrocarbures aromatiques chlorés et les hydrocarbures aliphatiques chlorés.

6. Procédé selon l'une des revendications 3 à 5, caractérisé en ce que le solvant aprotique apolaire utilisé est choisi parmi le tétrachlorure de carbone, le toluène, le benzène et le monochlorobenzène.

7. Procédé selon l'une des revendications 1 à 6, caractérisé en ce que la concentration initiale de la solution aqueuse d'acide nitrique est de 20 à 50% en poids d'acide nitrique par poids de solution.

8. Procédé selon l'une des revendications 1 à 7, caractérisé en ce que l'on opère entre 0°C et 100°C.

9. Procédé selon l'une des revendications 1 à 8, caractérisé en ce que l'on opère entre 20°C et 60°C.

10. Procédé de nitration selon l'une des revendications 1 à 9, caractérisé en ce qu'il est appliqué au dichloro-2,6 phénol en mélange avec du trichloro-2,4,6 phénol.

11. Procédé selon l'une des revendications 1 à 10, caractérisé en ce que l'on nitre le dichloro-2,6 phénol obtenu par chloration sélective du chloro-2 phénol, par le chlore gazeux, à une température comprise entre 40°C et 120°C, dans un solvant aprotique apolaire et en présence de 0,001% à 0,100% en poids d'une amine primaire, secondaire ou tertiaire par rapport au dit solvant.

12. Utilisation du dichloro-2,6 nitro-4 phénol selon l'une des revendications 1 à 11 pour préparer par hydrogénation catalytique ou hydrogénation chimique l'amino-4 dichloro-2,6 phénol.

13. Utilisation du dichloro-2,6 nitro-4 phénol selon l'une des revendications 1 à 11 pour préparer par méthylation de la fonction phénolique du dichloro-2,6 nitro-4 anisole.

## Ansprüche

1. Verfahren zur Nitrierung von 2,6-Dichlorphenol, dadurch gekennzeichnet, daß diese phenolische Verbindung in eine 10- bis 70gewichtsprozentige wäßrige Salpetersäurelösung eingebracht wird und daß sich das Gesamtmolverhältnis Salpetersäure/phenolische Verbindung (I) zwischen 10 und 1,2 befindet.

2. Verfahren nach Anspruch 1, dadurch gekennzeichnet, daß die phenolische Verbindung in festem Zustand in die wäßrige Salpetersäurelösung eingebracht wird.

3. Verfahren nach Anspruch 1, dadurch gekennzeichnet, daß die phenolische Verbindung in Form einer Lösung in ein mit Wasser unmischbares, apolares aprotisches Lösungsmittel eingebracht wird.

4. Verfahren nach Anspruch 3, dadurch gekennzeichnet, daß das apolare aprotische Lösungsmittel ein aliphatischer, ein cycloaliphatischer, ein aromatischer, ein chlorierter aliphatischer, ein chlorierter cycloaliphatischer oder ein chlorierter aromatischer Kohlenwasserstoff ist.

5. Verfahren nach Anspruch 3 oder 4, dadurch gekennzeichnet, daß das apolare aprotische Lösungsmittel aus den aromatischen, den chlorierten aromatischen und den chlorierten aliphatischen Kohlenwasserstoffen ausgewählt ist.

6. Verfahren nach einem der Ansprüche 3 bis 5, dadurch gekennzeichnet, daß das verwendete apolare aprotische Lösungsmittel aus Tetrachlorkohlenstoff, Toluol, Benzol und Monochlorbenzol ausgewählt ist.

7. Verfahren nach einem der Ansprüche 1 bis 6, dadurch gekennzeichnet, daß die Anfangskonzentration der wäßrigen Salpetersäurelösung 20 bis 50 Gew.% Salpetersäure, bezogen auf die Lösung, beträgt.

8. Verfahren nach einem der Ansprüche 1 bis 7, dadurch gekennzeichnet, daß man zwischen 0°C und 100°C arbeitet.

9. Verfahren nach einem der Ansprüche 1 bis 8, dadurch gekennzeichnet, daß man zwischen 20°C und 60°C arbeitet.

10. Verfahren zur Nitrierung nach einem der Ansprüche 1 bis 9, dadurch gekennzeichnet, daß es auf ein Gemisch von 2,6-Dichlorphenol mit 2,4,6-Trichlorphenol angewendet wird.

11. Verfahren nach einem der Ansprüche 1 bis 10, dadurch gekennzeichnet, daß man das 2,6-Dichlorphenol nitriert, das durch selektive Chlorierung des 2-Chlorphenols mit Chlorgas bei einer Temperatur zwischen 40°C und 120°C in einem apolaren aprotischen Lösungsmittel und in Gegenwart von 0,001 Gew.% bis 0,100 Gew.%, bezogen auf das Lösungsmittel, eines primären, sekundären oder tertiären Amins erhalten wird.

12. Verwendung des 2,6-Dichlor-4-nitrophenols nach einem der Ansprüche 1 bis 11 zur Herstellung von 4-Amino-2,6-dichlorphenol durch katalytische oder chemische Hydrierung.

13. Verwendung des 2,6-Dichlor-4-nitrophenols nach einem der Ansprüche 1 bis 11 zur Herstellung von 2,6-Dichlor-4-nitroanisol durch Methylierung der Phenolgruppe.

## Claims

1. Process for nitration of 2,6-dichlorophenol, characterized in that the said phenolic compound is introduced into an aqueous nitric acid solution having a concentration by weight of 10 to 70% and in that the overall mole ratio nitric acid/phenolic compound (I) is between 10 and 1.2.

2. Process according to Claim 1, characterized in that the phenolic compound is introduced in solid form into the aqueous nitric acid solution.

3. Process according to Claim 1, characterized in that the phenolic compound is introduced in the form of a solution in an apolar aprotic solvent which is immiscible with water.

4 . Process according to Claim 3, characterized in that the apolar aprotic solvent is an aliphatic hydrocarbon, a cycloaliphatic hydrocarbon, an aromatic hydrocarbon, a chlorinated aliphatic hydrocarbon, a chlorinated cycloaliphatic hydrocarbon or a chlorinated aromatic hydrocarbon.

5. Process according to one of Claims 3 and 4, characterized in that the apolar aprotic solvent is chosen from aromatic hydrocarbons, chlorinated aromatic hydrocarbons and chlorinated aliphatic hydrocarbons.

6. Process according to one of Claims 3 to 5, characterized in that the apolar aprotic solvent used is chosen from carbon tetrachloride, toluene, benzene and monochlorobenzene.

7. Process according to one of Claims 1 to 6, characterized in that the initial concentration of the aqueous nitric acid solution is 20 to 50% by weight of nitric acid per weight of solution.

8. Process according to one of Claims 1 to 7, characterized in that the operation is performed at between 0°C and 100°C.

9. Process according to one of Claims 1 to 8, characterized in that the operation is performed at between 20°C and 60°C.

10. Nitration process according to one of Claims 1 to 9, characterized in that it is applied to 2,6-dichlorophenol mixed with 2,4,6-trichlorophenol.

11. Process according to one of Claims 1 to 10, characterized in that the nitration is performed of 2,6-dichlorophenol, which is obtained by selective chlorination of 2-chlorophenol with gaseous chlorine at a temperature of between 40°C and 120°C in an apolar aprotic solvent and in the presence of 0.001% to 0.100% by weight, relative to the said solvent, of a primary, secondary or tertiary amine.

12. Use of the 2,6-dichloro-4-nitrophenol according to one of Claims 1 to 11 for preparing 4-amino-2,6-dichlorophenol by catalytic hydrogenation or chemical hydrogenation.

13. Use of the 2,6-dichloro-4-nitrophenol according to one of Claims 1 to 11 for preparing 2,6-dichloro-4-nitroanisole by methylation of the phenolic group.